# FASCICULE DE BREVET EUROPEEN

(11) **EP 1 023 603 B1**
(45) Date de publication et mention de la délivrance du brevet: **01.06.2005**
(21) Numéro de dépôt: 98947242.8
(22) Date de dépôt: 06.10.1998
(51) Int. Cl.: G01N 33/558, G01N 33/543

(54) **DISPOSITIF D'ESSAI ET PROCEDE POUR LA DETERMINATION D'ANALYTES DANS UN PRODUIT LAITIER LIQUIDE**
TESTVORRICHTUNG UND VERFAHREN ZUR BESTIMMUNG VON ANALYTEN IN EINEM FLÜSSIGEN MILCHPRODUKT
TEST DEVICE AND METHOD FOR DETERMINING ANALYTES IN A LIQUID DAIRY PRODUCT

(30) Priorité: 07.10.1997 BE 9700807; 25.06.1998 BE 9800485
(43) Date de publication de la demande: 02.08.2000
(73) Titulaire: UCB S.A., 1070 Bruxelles (BE)
(72) Inventeur: DEGELAEN, Jacques, B-1470 Genappes (BE); FRERE, Jean-Marie, B-4550 Nantrin (BE); GRANIER, Benoît, B-4130 Esneux (BE); JORIS, Bernard, B-4900 Spa (BE)
(74) Mandataire: Lechien, Monique
(86) Numéro de dépôt international: PCT/BE1998/000147
(87) Numéro de publication internationale: WO 1999/018439

(56) Documents cités:
- EP-A- 0 408 222
- EP-A- 0 806 666
- WO-A-93/18398
- WO-A-96/10177
- WO-A-96/15453
- US-A- 5 160 486
- US-A- 5 591 645

## Description

La présente invention se rapporte à un dispositif d'essai pour la détermination d'analytes dans un produit laitier liquide tel que le lait. Elle concerne également un procédé permettant la détection et la quantification d'analytes dans le lait grâce à ce dispositif d'essai ainsi qu'une trousse d'essai comprenant ce dispositif d'essai.

A l'heure actuelle, les exigences sanitaires de nombreux pays imposent de contrôler régulièrement la présence de diverses substances dans les produits laitiers, tels que médicaments vétérinaires et hormones, couramment utilisés dans l'élevage du bétail. Pour des raisons médicales évidentes, ces substances doivent être évitées dans les produits laitiers destinés à la consommation humaine.

Dans d'autres cas, il est souhaitable de disposer de tests permettant de détecter la présence de substances endogènes dans le lait afin d'optimiser les pratiques d'élevage du bétail. En particulier, la détermination rapide du taux d'hormones dans le lait permet d'identifier facilement les périodes propices à la reproduction.

Dans d'autres cas encore, on recherche des méthodes pratiques et fiables pour contrôler l'origine des produits laitiers dérivés des laits de diverses espèces animales. On recherche alors des méthodes qui permettraient d'identifier la présence de protéines caractéristiques du lait de certaines espèces par rapport à d'autres.

Par ailleurs, divers tests sont connus dans la littérature pour la détection d'analytes dans des liquides biologiques. Ces tests utilisent généralement des méthodes de détection faisant appel à un agent de reconnaissance (récepteur ou anticorps) qui reconnaît spécifiquement l'analyte ou un analogue de cet analyte, et à un agent de marquage (radioélément, enzyme, agent fluorescent, etc.) ci-après dénommés réactifs de détection. En fonction des éléments choisis, on parlera de radioimmunoessai (RIA), de radiorécepteuressai (RRA), d'enzymeimmunoessai (EIA) etc. Dans leur principe général, ces tests font appel à la combinaison minimale des deux éléments précités (réactifs de détection) qui permettra d'obtenir un résultat dont la valeur est une indication de la quantité d'analyte présent.

Il convient de remarquer qu'en fonction de la méthode de détection choisie, l'agent de marquage peut être couplé soit à l'agent de reconnaissance soit à l'analyte ou à une substance analogue de l'analyte du point de vue de sa reconnaissance par l'agent de reconnaissance. Il existe également des procédés dans lesquels l'agent de reconnaissance ou l'analyte ou la substance analogue de l'analyte contient intrinsèquement l'agent de marquage (par exemple un analyte marqué radioactivement).

Pour les produits laitiers. les tests de détection d'analytes les pius couramment décrits concernent la détection d'antibiotiques. Il est bien connu en effet d'utiliser des antibiotiques pour traiter certaines maladies infectieuses du bétail producteur de lait.

Or, pour des raisons médicales évidentes, le lait destiné à la consommation humaine doit, en principe, être exempt de toute trace d'antibiotiques. Par ailleurs, des concentrations en pénicilline de 0,005 U.I./ml ou moins peuvent avoir des incidences néfastes lors de la fabrication de produits dérivés du lait comme le fromage. le yaourt, etc.

Plusieurs situations peuvent être envisagées. Dans un premier cas, par exemple pour détecter la présence d'antibiotiques à la ferme avant de transvaser dans un camion, la priorité sera donnée à un test extrêmement rapide (inférieur à 5 minutes) et simple. On peut également envisager d'utiliser un tel test rapide lorsque par exemple l'antibiotique qui a été utilisé pour le traitement est connu et que par ailleurs ce test permet la détection de l'antibiotique en question à la norme légale. Dans le deuxième cas, lorsque l'accent n'est pas mis sur la rapidité, l'importance est de détecter la majorité, si pas tous les antibiotiques aux normes légales.

La législation de certains pays impose en effet des normes de qualité bien précises. Par exemple, les autorités américaines exigent que les concentrations dans le lait des six antibiotiques suivants ne dépassent pas des valeurs bien précises :
pénicilline, 5 ppb; ampicilline, 10 ppb; amoxicilline, 10 ppb; cloxacilline, 10 ppb; céphapirine, 20 ppb; ceftiofur, 50 ppb. L'Union Européenne impose, elle aussi, des normes de qualité: pénicilline 4 ppb; amoxicilline 4 ppb; ampicilline 4 ppb; cloxacilline 30 ppb; dicloxacilline 30 ppb; oxacilline 30 ppb; céphapirine 10 ppb; ceftiofur 100 ppb: cefquinone 20 ppb; nafcilline 30 ppb; céfazoline 50 ppb.

Il pourrait donc être intéressant de disposer d'un test qui permettrait de détecter la majorité des antibiotiques. Par ailleurs, au niveau de l'industrie laitière, on peut considérer qu'à défaut d'avoir un test présentant toutes les caractéristiques de rapidité, de sensibilité et de simplicité, un test qui permettrait d'allier au mieux ces trois paramètres, même s'ils ne sont pas totalement couverts, serait intéressant.

Le brevet US 4,239,852 décrit un procédé microbiologique pour la détection d'antibiotiques à noyau β-lactame dans le lait. Selon ce procédé, l'échantillon de lait est incubé, d'une part, en présence de parties de cellules d'un micro-organisme très sensible aux antibiotiques, en particulier Bacillus stearothermophilus, et, d'autre part, en présence d'un antibiotique marqué par un élément radioactif ou par une enzyme. L'incubation est menée dans des conditions permettant aux antibiotiques éventuellement présents dans l'échantillon et à l'antibiotique marqué de se lier aux parties de cellules.

Après incubation. les parties de cellules sont séparées du mélange, puis lavées. Ensuite, la quantité d'antibiotique marqué lié aux parties de cellules est déterminée et comparée à un standard. La quantité d'antibiotique marqué lié aux parties de cellules est inversement proportionnelle à la concentration d'antibiotique présent dans l'échantillon de lait analysé.

Ce procédé requiert des manipulations relativement délicates, notamment lors de la séparation des parties de cellules du mélange. En outre, dans sa version la plus sensible, ce procédé utilise un antibiotique marqué par un élément radioactif ( ¹⁴C ou ¹²⁵I). Dans ce cas, la détermination de la quantité d'antibiotique éventuellement présent dans le lait nécessite d'avoir recours à un appareil spécial, comme par exemple un compteur à scintillation. De plus, la manipulation de produits radioactifs, même en très faibles quantités n'est pas totalement exempte de danger pour la personne qui effectue l'analyse.

La demande de brevet européen 593.112 décrit une autre méthode permettant la détection d'antibiotiques dans le lait. Cette méthode utilisé une protéine isolée à partir d'un microorganisme sensible aux antibiotiques, tel que Bacillus stearochermophilus. Cette protéine est en outre marquée par une enzyme telle qu'une peroxydase.

Le test procède de la manière suivante: un échantillon de lait est incubé dans un tube en présence de la protéine marquée; après incubation, le lait est transféré dans un second tube sur les parois duquel un antibiotique de référence a été immobilisé; on procède à une seconde incubation, puis le contenu du tube est éliminé; les parois de ce second tube sont lavées trois fois au moyen d'une solution de lavage, qui est elle-même éliminée, puis les résidus présents dans le second tube sont transférés sur un papier absorbant; on ajoute alors un substrat colorant dans le second tube qui est incubé une nouvelle fois, puis on ajoute une solution arrêtant le développement de la couleur; la coloration du tube est comparée à la coloration d'un test identique effectué parallèlement sur un échantillon standard d'antibiotique. La quantité de protéine marquée immobilisée sur le support, et donc, l'intensité de la coloration, est inversement proportionnelle à la quantité d'antibiotique présent dans l'échantillon de lait analysé.

Selon l'exemple 1 de cette demande de brevet, ce test permet de détecter la pénicilline G jusqu'à des concentrations de l'ordre de 5 ppb et permet la détection de l'amoxicilline (5 ppb), de l'ampicilline (10 ppb), de la cephapirine (5 ppb) et du ceftiofur (5 ppb).

Toutefois, le test est assez fastidieux à mettre en oeuvre, en particulier par du personnel non qualifié. En effet, ce test comporte de nombreuses étapes, y compris des étapes de transvasement de liquide et de résidus et plusieurs étapes de rinçage. Etant donné le nombre et le type d'étapes requises dans ce test, l'obtention d'un résultat fiable dépend fortement du savoir-faire expérimental de l'exécutant.

En outre, l'interprétation du résultat nécessite de faire deux tests en parallèle, ce qui multiplie et complique encore les opérations.

On connaît également d'autres types de procédés enzymatiques permettant de déterminer de faibles concentrations d'antibiotiques dans le lait (J.M. FRERE et al., Antimicrobial Agents and Chemotherapy, 18(4), 506-510 (1980) ainsi que les brevets EP 85.667 et EP 468.946) qui sont basés sur l'utilisation d'une enzyme spécifique, en l'occurrence, la D-alanyl-D-alanine-carboxypeptidase exocellulaire soluble, qui est produite par Actinomadura R39 (ci-après désignée "enzyme R39"). L'enzyme R39 possède une activité spécifique d'hydrolyse des groupements D-alanyl-D-alanine de divers peptides et est également capable d'hydrolyser des thioesters particuliers.

En outre, l'enzyme R39 réagit avec les antibiotiques à noyau β-lactame pour former très rapidement un complexe enzyme-antibiotique équimolaire, inactif et sensiblement irréversible.

Dans la version la plus récente de ce test (EP 468.946), un volume déterminé d'un échantillon du liquide à examiner est incubé avec une quantité prédéterminée de l'enzyme R39 dans des conditions permettant à l'antibiotique β-lactame éventuellement présent dans l'échantillon de réagir avec l'enzyme pour former un complexe enzyme-antibiotique équimolaire, inactif et sensiblement irréversible.

Ensuite, une quantité déterminée de substrat de type thioester est incubée avec le produit obtenu au premier stade dans des conditions permettant l'hydrolyse du substrat par l'enzyme R39 résiduelle qui n'a pas été complexée avec l'antibiotique lors de la première incubation. La quantité d'acide mercaptoalcanoïque ainsi formée est alors déterminée par dosage colorimétrique à l'aide d'un réactif capable de produire une coloration par réaction avec le groupe SH libre de l'acide mercaptoalcanoïque. L'intensité de la coloration est comparée à un étalon, préalablement établi à partir d'échantillons contenant des quantités connues d'antibiotiques. La détermination quantitative peut se faire par mesure au spectrophotomètre; dans le cas du lait, la clarification préalable de l'échantillon peut s'avérer nécessaire.

Clairement, ce test comporte moins d'étapes et est plus simple à mettre en oeuvre que le test décrit dans la demande de brevet EP 593.112. Toutefois, il est limité à la détection d'antibiotiques à noyau β-lactame, et ce, jusqu'aux seuils limites de détection accessibles avec l'enzyme R39. Tel quel, ce test ne peut pas être utilisé avec d'autres récepteurs d'antibiotiques, et ne peut pas directement servir de base pour 1a détection d'autres analytes dans le lait. EP 0 408 222 décrit un dispositif permettant de séparer des composants compris dans un liquide, ce dispositif comprend une membrane de séparation microporeuse.

Puisque les tests actuels présentent encore divers inconvénients, la demanderesse s'est fixé comme objectif de rechercher de nouvelles méthodes pour la détection d'analytes dans les produits laitiers liquides. les méthodes recherchées devant permettre la détermination fiable de différents types d'analytes, de préférence au moment de la collecte à la ferme. La demanderesse a donc recherché une méthode qui permette très rapidement d'obtenir un résultat fiable et sensible en un nombre limité d'étapes simples, ne requérant pas de savoir-faire expérimental particulier. En outre, la demanderesse a recherché une méthode fournissant un résultat facilement détectable visuellement et pouvant en outre faire l'objet d'une quantification par mesure instrumentale.

La demanderesse vient maintenant de découvrir que ces objectifs peuvent être atteints gràce à l'utilisation d'un nouveau dispositif d'essai permettant de déterminer aisément la présence d'analytes dans les produits laitiers liquides, en particulier, le lait.

C'est pourquoi la présente invention concerne un dispositif d'essai permettant de détecter la présence d'analytes dans un produit laitier liquide par migration capillaire tangentielle dudit produit laitier. Le dispositif d'essai selon l'invention comprend un support solide (1) qui possède une première et une seconde extrémité, sur lequel sont fixées, successivement, en partant de la première extrémité,
- une membrane (2) permettant la purification du liquide analysé,
- une membrane (3) sur laquelle une ou plusieurs substances de capture sont immobilisées, et
- une membrane (4) absorbante,
caractérisé en ce que la membrane (2) est une membrane Leukosorb fabriquée à partir de fibres de polyester non-tissées et est capable de retenir les substances présentes dans le produit laitier, qui empêchent la migration sur le dispositif d'essai des analytes éventuellement présents dans le produit laitier et des réactifs de détection utilisés selon la méthode pratiquée, et ce, lors de la migration capillaire tangentielle de l'échantillon après trempage de la première extrémité du dispositif d'essai dans le produit laitier analysé.

Selon un mode d'implémentation particulier de l'invention, le dispositif d'essai selon la présente invention possède en outre une membrane (5) sur laquelle au moins un réactif de détection a été déposé, ce réactif de détection étant capable de se solubiliser rapidement en présence dudit produit laitier. Selon ce mode d'implémentation particulier, la membrane (5) doit être située avant la membrane (3). Elle peut être située, par exemple, soit avant la membrane (2) à la première extrémité du dispositif, soit entre la membrane (2) et la membrane (3) ou encore en dessous ou au dessus de la membrane (2).

Les différentes membranes présentes dans le dispositif d'essai selon la présente invention se superposent à leurs extrémités de manière à assurer la migration continue du produit laitier d'une zone à l'autre. De préférence, la membrane (3) est placée de manière à ce que son extrémité proximale soit placée en dessous de la membrane (2) et son. extrémité distale en dessous de la membrane (4). Les membranes peuvent éventuellement être maintenues en contact les unes avec les autres grâce à un film plastique adhésif (6). Dans ce cas, le film plastique adhésif est choisi de manière à ne pas affecter la migration du liquide sur le dispositif d'essai.

L'option de couvrir le dispositif d'essai par un film plastique adhésif présente deux avantages: il assure un contact parfait à la superposition des membranes et constitue un film protecteur. Le film plastique adhésif (6) peut soit recouvrir complètement les membranes (2), (3), (4) et (5), soit recouvrir partiellement les membranes individuelles. De préférence, le film plastique adhésif (6) ne recouvre pas les premiers millimètres de la première extrémité de manière à permettre une migration plus rapide du liquide sur la membrane (2) du dispositif d'essai.

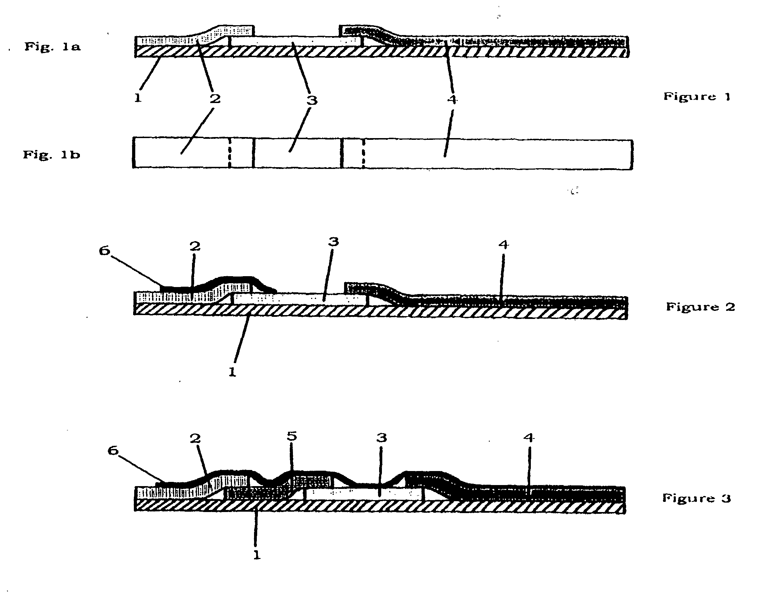

Les Figures 1 à 3 illustrent des exemples de dispositifs d'essai selon la présente invention. Les Figures la, 2 et 3 présentent des vues frontales et la Figure 1b présente une vue en section longitudinale.

Le support solide (1) présent dans le dispositif d'essai selon la présente invention est en verre ou en matière plastique, de préférence, en matière plastique. Dans le cas d'un support en matière plastique, son épaisseur est généralement comprise entre 0,05 et 1 mm, de préférence entre 0,1 et 0,6 mm. Les membranes sont fixées sur le support solide (1) grâce à un adhésif.

La membrane (2) peut être de différents types. D'une part, elle doit être capable de retenir les substances présentes dans le produit laitier qui empêchent la migration sur le dispositif d'essai des analytes éventuellement présents dans le produit laitier et des réactifs de détection utilisés selon la méthode pratiquée. D'autre part, elle doit permettre la migration rapide des analytes et réactifs de détection sur le dispositif d'essai, tout en préservant l'activité de ces analytes et réactifs de détection lors de cette migration. Un exemple non limitatifs de membranes permettant d'obtenir ce rèsultat est Leukosorb® LK4 (disponibles chez Pall Gelman Sciences).

La membrane Leukosorb® utilisée de préférence est une membrane fabriquée à partir de fibres de polyester non tissées, destinée à la rétention de leukocytes à partir d'échantillons cliniques issus du sang, de l'urine, de la salive et du liquide cérébrospinal. La rétention des leukocytes est réalisée par filtration du fluide, par passage transversal à travers la membrane.

La demanderesse a découvert de façon inattendue que ces types de membranes permettent également d'assurer une fonction très importante pour la détection d'analytes dans un produit laitier grâce aux dispositifs d'essai selon la présente invention, à savoir, la rétention des substances présentes dans les produits laitiers, qui empêchent le bon déroulement d'un test de détection par migration capillaire tangentielle du produit laitier sur lesdits dispositifs d'essai, tout en permettant la migration rapide des analytes et réactifs de détection.

Pour opérer cette fonction de manière optimale, la membrane (2) doit être suffisamment longue pour permettre l'élimination de toutes les substances présentes dans le produit laitier qui empêchent la migration rapide des analytes et réactifs de détection sur le dispositif d'essai.

La membrane (3) doit permettre d'immobiliser une ou plusieurs substances de capture et doit permettre la migration rapide de l'échantillon de produit laitier après passage sur la membrane (2). De préférence, la membrane (3) est consolidée sur un support non poreux de type polyester. Des exemples non limitatifs de membranes convenant selon la présente invention sont les membranes en nitrocellulose à vitesse capillaire élevée telles que les membranes Hi-Flow (disponibles chez Millipore), de préférence les membranes Hi-Flow de type SX ou ST. Ces membranes procurent un résultat optimal en combinaison avec les membranes (2) telles qu'identifiées précédemment.

Une ou plusieurs substances de capture sont immobilisées sur la membrane (3). Le type de substance de capture immobilisée dépend bien entendu de la méthode pratiquée pour la détection des analytes; les substances de capture sont capables d'immobiliser sélectivement au moins un des constituants présents dans le liquide migrant sur le dispositif d'essai, tel que l'un des réactifs de détection ou le produit résultant de la formation d'un complexe entre l'analyte ou une substance analogue de cet analyte et au moins un réactif de détection ou encore, le produit résultant de la formation d'un complexe entre plusieurs réactifs de détection. La substance de capture peut aussi être un des réactifs de détection. Les substances de capture sont placées de manière concentrée sur une portion ou plusieurs portions bien délimitées de la membrane (3), telles que des disques ou des bandes minces, ou tout autre motif approprié pour l'application. Quel que soit le motif choisi, il doit permettre d'obtenir une lecture claire du résultat.

Quant à sa dimension, la membrane (3) doit être d'une longueur suffisante pour contenir toutes les substances de capture utilisées, et ce, dans l'ordre et dans les quantités requises selon la méthode de détection pratiquée.

Le type de membrane utilisé pour la membrane (4) importe peu, pour autant que celle-ci soit capable d'absorber et stocker le liquide après son passage sur les membranes précédentes. La membrane (4) est de dimension suffisamment grande pour permettre d'absorber le liquide après son passage sur la membrane (3), mais aussi, d'un point de vue pratique, pour permettre une prise manuelle plus aisée du dispositif d'essai.

Eventuellement, le dispositif d'essai selon la présente invention peut comprendre une membrane (5) sur laquelle au moins un réactif de détection a été déposé. La membrane (5) doit permettre la migration du produit laitier, tout en permettant la solubilisation et le relargage du (ou des) réactif(s) de détection lors du passage du flux de produit laitier. Des exemples non limitatifs de membranes pouvant convenir à cet effet. sont: les membranes à base de résines en fibre de verre telles que les membranes T5NM (disponibles chez Millipore), les membranes F075-14 ou F075-17 ou GF/C (disponibles chez Whatman) la membrane Cytosep 1663 (disponible chez Pall Gelman Sciences), les membranes à base de résines en fibres de polyester telles que les membranes Accuwick (disponible ches Pall Gelman Sciences) le papier cellulosique 3 mm (disponibles chez Whatman) ou encore les membranes de type Release Matrix PT-R2 (disponibles chez Advances Micro Devices). De préférence, on utilise une membrane à base de résines en fibres de polyester telle que les membranes Accuwick. La membrane (5) a une longueur suffisante pour supporter la quantité de réactif de détection désirée.

Les dispositifs d'essai selon la présente invention sont fabriqués par les méthodes connues de l'homme du métier. On peut par exemple préparer des cartes grâce a des laminateurs disponibles dans le commerce. Les substances de capture utilisées sont déposées sur la membrane (3) sous la forme de solutions, avant ou après l'assemblage des cartes. Le dépôt de ces solutions peut être réalisé de manière très précise grâce à des appareillages disponibles dans le commerce, tel que le Dispenser X-Y Platform Biojet Quanti-3000 de Bio Dot, Inc. Ces solutions déposées sont aussitôt évaporées, par exemple, en plaçant la carte sous un flux d'air chaud. Pour la production à grande échelle, on peut également préparer des rouleaux. Ensuite, les cartes et rouleaux portant les substances de capture désirées sont découpées en lamelles, chacune de ces lamelles constituant un dispositif d'essai selon l'invention.

Lorsque le dispositif d'essai comprend une membrane (5), le(s) réactif(s) de détection peut(vent) y être déposé(s) avant l'assemblage des cartes ou rouleaux, par simple immersion de la membrane (5) dans une solution contenant le(s) réactif(s) de détection. Alternativement, il peut(vent) être déposé(s) après assemblage des cartes ou rouleaux par une technique analogue à celle utilisée pour le dépôt des substances de capture sur la membrane (3).

Dans une variante de l'invention, le dispositif est placé dans un boitier plastique qui présente deux ouvertures: la première, en forme de cuvette, est placée juste au-dessus de la membrane (2), et permet de recevoir le liquide à analyser; la seconde est une fenêtre d'ouverture permettant de visualiser le résultat sur la membrane (3). Dans ce cas, le dispositif d'essai ne comprend pas de film plastique adhésif (6).

Le dispositif d'essai selon la présente invention permet de détecter la présence d'analytes dans un produit laitier liquide, en particulier le lait.

C'est pourquoi la présente invention concerne également un procédé pour la détection d'analytes dans un produit laitier liquide, utilisant un dispositif d'essai selon la présente invention ainsi que des réactifs de détection, comprenant les étapes suivantes:
a) la mise en contact d'un volume déterminé de produit laitier avec le dispositif d'essai selon la présente invention, cette mise en contact ayant lieu à la première extrémité du dispositif d'essai,
b) la migration tangentielle par capillarité du produit laitier sur le dispositif d'essai de manière à ce que les analytes et les réactifs de détection éventuellement présents dans le produit laitier passent progressivement sur la membrane (2), puis la membrane (3), et de manière à ce que les constituants du produit laitier qui ne sont pas arrêtés par les membranes (2) et (3) aboutissent dans la membrane (4), et
c) la détermination d'une fixation sur la membrane (3).

Le procédé selon la présente invention permet de détecter des analytes dans un produit laitier liquide, par exemple du lait, du petit lait, du lait battu, ... La présente invention s'adresse plus particulièrement à la détection d'analytes tels que médicaments vétérinaires, hormones ou protéines susceptibles d'être présents dans le lait.

Les réactifs de détection utilisés selon le procédé de l'invention peuvent varier en nombre et en nature en fonction du mode d'implémentation de l'invention, lui-même basé sur la méthode de détection pratiquée. Le procédé selon l'invention utilise au moins deux réactifs de détection. Le premier réactif de détection est un agent de reconnaissance capable de reconnaitre spécifiquement l'analyte, et sera ci-après dénommé "Identifiant". Le second réactif de détection est un agent de marquage, et sera ci-après dénommé "Marqueur". Il convient de remarquer qu'en fonction du mode d'implémentation choisi, certains réactifs de détection peuvent être présents sur la membrane (3) ou sur la membrane (5). En fonction de l'analyte à détecter et des réactifs de détection utilisés, il peut s'avérer nécessaire d'ajouter un ou plusieurs réactifs de détection avant l'étape de mise en contact du produit laitier avec le dispositif d'essai selon l'invention et de maintenir ce mélange dans des conditions d'incubation permettant la formation d'un complexe entre des réactifs de détection et l'analyte ou une substance analogue de l'analyte. En fonction de la méthode choisie, l'Identifiant et le Marqueur peuvent être couplés entre eux ou peuvent n'être qu'une seule et même substance. D'autre part, il peut y avoir plusieurs Identifiants et/ou Marqueurs.

L'Identifiant permet de détecter la présence du type d'analyte recherché grâce à sa capacité à reconnaitre spécifiquement cet analyte ou une substance analogue de cet analyte. Il peut s'agir d'un récepteur capable de former sélectivement un complexe stable et essentiellement irréversible avec l'analyte ou une substance analogue de l'analyte ou d'un anticorps monoclonal ou polyclonal spécifique de l'analyte ou d'une substance analogue de l'analyte. Pour la détection d'antibiotiques, l'Identifiant peut être choisi parmi des anticorps monoclonaux ou polyclonaux spécifiques ou parmi les récepteurs obtenus à partir de microorganismes sensibles aux antibiotiques. tels que les récepteurs obtenus à partir des espèces Bacillus (Bacillus stearothermonhilus, Bacillus subtilis, Bacillus licheniformis, ...) Streptococcus (Streptococcus thermophilus, ...), Actinomvcètes (Actinomadura R39, ...).

Selon un mode de réalisation préféré de l'invention, on utilise un Identifiant qui comprend un récepteur sensible aux antibiotiques à noyau β-lactame obtenu à partir de Bacillus licheniformis tel que le récepteur BlaR ou le récepteur BlaR-CTD. L'isolement et la séquence peptidique de la protéine BlaR sont décrits dans Y. ZHU et al., J. Bacteriol., 1137-1141 (1990); le récepteur BlaR-CTD est le domaine carboxy terminal de BlaR dont l'isolement et la séquence peptidique sont décrits dans B. JORIS et al., FEMS Microbiology Letters, 107-114 (1990).

L'utilisation des récepteurs BlaR ou BlaR-CTD selon la présente invention pour la détection d'antibiotiques à noyau β-lactame présente des avantages importants par rapport aux agents de reconnaissance utilisés jusqu'à présent. En effet, les récepteurs BlaR et BlaR-CTD sont capables de complexer très rapidement un nombre élevé d'antibiotiques, et ce, à une température d'incubation inférieure à celle nécessitée pour les agents de reconnaissance connus tels que, par exemple, les récepteurs obtenus à partir de Bacillus stearothermophilus.

Le second type de réactif de détection utilisé est un Marqueur qui permet de visualiser et de quantifier directement ou indirectement la présence des analytes dans le produit laitier. Les Marqueurs utilisables selon l'invention peuvent être particulaires, fluorescents, radioactifs, luminescents ou enzymatiques. On choisit de préférence un Marqueur particulaire qui fournit un signal visuel aisément détectable, même lorsque celui-ci est présent en faible quantité. A titre d'exemples non limitatifs, on citera les particules colloïdales métalliques (platine, or, argent, etc.), les particules colloïdales de sélénium, carbone, soufre, tellure ou encore les particules colloïdales de latex synthétiques colorés. Les particules d'or colloïdal ayant un diamètre particulaire compris entre 1 et 60 nm sont particulièrement préférées: elles fournissent une coloration rose-rouge intense aisément détectable.

Le Marqueur permet de déterminer la présence de l'analyte dans l'échantillon de produit laitier grâce à son couplage à un ou plusieurs réactifs de détection. à l'analyte ou à une substance analogue de l'analyte.

Le couplage entre le Marqueur et le réactif de détection peut être réalisé selon les méthodes connues de l'homme du métier. Lorsqu'on utilise un Marqueur particulaire, le marquage peut avoir lieu soit par adsorption directe sur les particules, soit indirectement, par le biais d'un bras d'ancrage chimique, tel qu'un complexe biotine/anti-biotine, par exemple. Ce couplage peut avoir lieu, soit avant l'étape de mise en contact du produit laitier avec le dispositif d'essai selon l'invention. soit pendant la migration du produit laitier sur le dispositif d'essai selon l'invention.

Selon un mode d'implémentation particulier de l'invention, on utilise un troisième type de réactif de détection, ci-aprés dénommé "Référence". Il s'agit d'une substance ajoutée en quantité connue à l'échantillon analysé, et qui se fixe sur une substance de capture spécifique immobilisée sur la membrane (3). La Référence fournit une bande dont l'intensité sert de référence pour la quantification de l'analyte.

En ce qui concerne la mise en contact du produit laitier avec le dispositif d'essai selon l'invention (étape a) du procédé), elle est réalisée en plaçant le dispositif d'essai selon la présente invention dans un récipient au fond duquel se trouve l'échantillon à analyser. Le dispositif d'essai est placé essentiellement verticalement dans le récipient, de manière à ce que la première extrémité du dispositif soit en contact avec le mélange.

Dans la variante de l'invention utilisant un dispositif d'essai placé dans un boitier plastique, celui-ci est disposé horizontalement et la mise en contact s'effectue en déposant un aliquot d'échantillon à analyser dans l'ouverture en forme de cuvette placée au-dessus de la membrane (2).

Pour l'étape de migration b), on laisse migrer le liquide par capillarité sur le dispositif d'essai selon l'invention. Le liquide qui migre par capillarité sur le dispositif d'essai selon l'invention rencontre tout d'abord la membrane (2) qui permet de retenir les substances présentes dans le produit laitier qui empêchent la migration des analytes éventuellement présents dans le produit laitier et réactifs de détection sur le dispositif d'essai. Les analytes et les réactifs de détection migrent ensuite sur la membrane (3) sur laquelle une ou plusieurs substances de capture ont été immobilisées. Les substances de capture immobilisent sélectivement au moins un des constituants présents dans le liquide analysé. Selon un mode d'implémentation particulier de l'invention, on utilise une substance de capture placée en fin de parcours de migration du liquide sur la membrane (3) qui est capable de fixer tous les Marqueurs qui n'ont pas été arrêtés par les substances de capture précédentes. Cette substance de capture permet de compléter les informations quantitatives fournies par les substances de capture précédentes.

La détermination d'une fixation sur la membrane (3) (étape c) du procédé) s'effectue simplement en déterminant la présence de Marqueurs dans cette zone. Cette détermination est possible simplement visuellement. Toutefois, si l'on désire avoir une mesure précise de l'intensité des signaux observés, on peut avoir recours à un appareil capable de mesurer l'intensité du signal observé. Lorsqu'on utilise une Référence, elle est fixée par une substance de capture spécifique qui fournit une référence interne pour la mesure de l'intensité des signaux observés.

L'interprétation du résultat obtenu dépend de la méthode de détection pratiquée, à savoir, des réactifs de détection et substances de capture utilisées.

Par rapport aux procédés de détection d'analytes dans le lait précédemment décrits dans la littérature, le procédé selon la présente invention présente les avantages suivants. En premier lieu, ce procédé est très rapide et extrêmement simple à mettre en oeuvre: il comporte essentiellement deux étapes de manipulation aisée, ne requérant pas de savoir-faire expérimental particulier. Ensuite, l'appréciation qualitative et quantitative du résultai est immédiate et ne nécessite pas d'opérations supplémentaires particulières, telles que celles requises lorsque la détection est effectuée par des colorants et/ou marqueurs enzymatiques. En outre, ce procédé est directement applicable pour la détection de différents types d'analytes. Enfin, dans le mode d'implémentation utilisant une Référence. le résultat est directement interprétable et quantifiable, sans qu'il soit nécessaire d'avoir recours à un ou plusieurs tests de référence. La présente invention concerne aussi un procédé tel que défini dans les revendications 14, 15 et 19.

La présente invention concerne aussi une trousse d'essai pour la détection d'analytes dans un produit laitier comprenant un dispositif d'essai selon la présente invention. Le cas échéant, 1a trousse d'essai selon la présente invention peut également contenir les réactifs de détection à ajouter à l'échantillon avant la mise en contact du produit laitier avec le dispositif d'essai.

Les exemples qui suivent illustrent différents aspects et modes d'implémentation de la présente invention sans toutefois en limiter sa portée.

### Exemple 1. Fabrication des dispositifs d'essai. Modes opératoires généraux.

### 1.1. Assemblage de cartes membranaires.

Des cartes ayant une taille de 300 x 76,2 mm sont d'abord assemblées au moyen d'un laminateur de type Chlamshell Laminator (disponible chez Bio Dot, Inc.) selon la méthode suivante:

On découpe un rectangle de support plastique de type ArCare 8565 (disponible chez Adhesive Research) de dimension 300 x 76,2 mm (support solide (1)). On découpe ensuite un rectangle de membrane Leukosorb® LK4 (disponible chez Pall Gelman Sciences) de dimension 300 x 20 mm (membrane (2)), un rectangle de membrane Hi-Flow SX (disponible chez Millipore) de dimension 300 x 25 mm (membrane (3)), un rectangle de membrane de cellulose 3 mm (disponible chéz Whatmann) de dimension 300 x 40 mm (membrane (4)) et un rectangle de membrane Accuwick (disponible chez Pall Gelman Sciences) de dimension 300 x 0,8 mm (membrane (5)).

On loge successivement les membranes (2) et (4), puis (5), puis (3) dans un emplacement spécifique du moule inférieur du laminateur. Le support solide (1) recouvert d'adhésif est quant à lui maintenu dans le couvercle de l'appareil, le côté adhésif étant exposé à l'air. Les membranes logées dans le moule inférieur sont mises en contact avec le support adhésif en refermant le laminateur; les membranes sont maintenues exactement en place au moyen d'une succion d'air réalisée par une pompe à vide. Lorsque le vide est rompu, on récupère une carte constituée du support solide (1) sur lequel les membranes (2), (3), (4) et (5) sont fixées.

### 1.2. Dépôt des substances de capture sur la membrane (3).

Le dépôt des substances de capture sur la membrane (3) est effectué avant ou après l'assemblage selon l'exemple 1.1.

On prépare une solution aqueuse contenant la substance de capture. Elle est déposée sur la membrane (3) de la carte membranaire préparée à l'exemple 1.1. au moyen d'un "Dispenser" de type X-Y Platform Biojet Quanti-3000 de Bio Dot, Inc.

Les solutions déposées sont immédiatement évaporées en plaçant l'ensemble de la carte pendant une minute sous un air pulsé chaud à 60°C.

### 1.3. Dépôt de la substance de marquage sur la membrane (5).

### a) Avant l'assemblage selon l'exemple 1.1.

On prépare une solution aqueuse contenant la substance de marquage. La membrane (5) est immergée dans cette solution. Elle est ensuite égouttée, puis séchée une nuit à température ambiante sous un vide de 0,5 bar.

### b) Après l'assemblage selon l'exemple 1.1.

On procède selon le mode opératoire décrit à l'exemple 1.2. pour le dépôt des substances de capture.

### 1.4. Dépôt du film plastique adhésif (6).

On découpe un rectangie de film adhésif du type ArCare 7759 (disponible chez Adhesive Researchl de dimension 300 x 20 mm pour un recouvrement partiel, et 300 x 71.2 mm pour un recouvrement de toutes les membranes.

La carte obtenue selon l'exemple 1.1. est placée dans le moule inférieur d'un laminateur et le film adhésif est placé dans le couvercle du laminateur, le côté adhésif étant exposé à l'air. Le film plastique adhésif est mis en contact avec la carte membranaire lors de la fermeture de l'appareil.

### 1.5. Découpe en lamelles.

Les cartes obtenues après assemblage sont découpés en lamelles à l'aide d'un appareil de type guillotine ou à l'aide d'un appareil rotatif (disponible chez Bio Dot, Kinematic ou Akzo). Les lamelles des extrémités sont écartées. les autres lamelles étant prêtes à l'emploi.

Pour leur conservation, les dispositifs d'essai sont placés dans un récipient opaque et hermétique en présence d'un agent dessicant (Silgelac, France).

### 1.6. Présentation dans un carter plastique.

On place le dispositif d'essai dans un boîtier plastique qui présente deux ouvertures: la première, en forme de cuvette, est placée juste au-dessus de la membrane (2), et permet de recevoir le liquide à analyser; la seconde est une fenêtre d'ouverture permettant de visualiser le résultat sur la membrane (3).

### Exemple 2. Détection d'antibiotiques à noyaux β-lactame dans le lait au moyen de l'enzyme R39.

### 2.1. Identifiant.

L'identifiant utilisé dans cet exemple est La D-alanyl-D-alanine-carboxypeptidase exocellulaire soluble produite par Actinomadura R39 obtenue selon le. mode opératoire décrit dans J.-M. FRERE et al., Antimicrobial Agents and Chemotherapy, 18(4), 506-510(1980).

### 2.2. Couplage de l'Identifiant au Marqueur.

### 2.2.1. Biotinylation de l'Identifiant.

250 µl d'une solution aqueuse d'enzyme R39 à 1 mg/ml sont dialysés pendant 24 heures contre 500 ml de tampon HNM (Hepes 10 mM, pH 8, NaCl 10 mM, MgCl₂ 5 mM). On ajoute alors à cette solution d'enzyme R39 dialysée, 2 ml de tampon bicarbonate (0,1 M en bicarbonate de sodium, pH 9) et 250 µl d'une solution d'ester 6-(biotinamido)caproïque de N-hydroxy-succinimide à 5 mg/ml dans de la DMF anhydre. Cette solution est agitée doucement sur agitateur pour tubes sur axe rotatif de type LABINCO (disponible chez VEL, Belgique) à une vitesse de 2 tours/minute pendant 3 heures à température ambiante et à l'abri de la lumière. La solution ainsi obtenue est dialysée contre du tampon HNM (Hepes 100 mM; pH 8, NaCl 100 mM, MgCl₂ 50 mM) pendant 24 heures. De cette manière, on obtient une solution d'enzyme R39 biotinylée que l'on dilue dans du tampon HNM-BSA (Hepes 500 mM; pH 8, NaCl 500 mM, MgCl₂ 250 mM, BSA 10 mg/ml) jusqu'à une concentration de 100 µg d'enzyme R39 par ml de tampon. Cette solution est stockée à -20°C.

### 2.2.2. Marqueur.

Comme Marqueur, on utilise des particules d'or ayant un diamètre de 40 nm, sur lesquelles un anticorps de chèvre anti-biotine a été déposé sous la forme de suspensions dans une solution aqueuse de tétraborate de sodium à 2 mM à pH 7,2. stabilisée par de l'azoture de sodium à 0.1% (disponible chez BRITISH BIOCELL (Réf. GAB40). La densité optique de ces suspensions à 520 nm est d'environ 10 et la concentration en protéine est d'environ 24 µg/ml.

### 2.2.3. Couplage de l'Identifiant biotinylé au Marqueur.

### Solution A pour test rapide

La solution d'enzyme R39 biotinylée préparée à l'exemple 2.2.1. est diluée 25 fois par du tampon HNM-BSA (Hepes 500 mM, pH 8, NaCl 500 mM, MgCl₂ 250 mM, BSA 10 mg/ml). On mélange à température ambiante 17,5 parties en volume de cette solution diluée d'enzyme R39 biotinylée, 9,27 parties en volume de suspension de particules d'or servant à marquer l'enzyme R39 et 6 parties en volume de suspension de particules d'or de référence (voir exemple 2.3).

### Solution B pour test sensible

La solution d'enzyme R39 biotinylée préparée à l'exemple 2.2.1. est diluée 50 fois par du tampon HNM-BSA (Hepes 500 mM, pH 8, NaCl 500 mM, MgCl₂ 250 mM, BSA 10 mg/ml). On mélange à température ambiante 17,5 parties en volume de cette solution diluée d'enzyme R39 biotinylée, 9,27 parties en volume de suspension de particules d'or servant à marquer l'enzyme R39 et 6 parties en volume de suspension de particules d'or de référence (voir exemple 2.3).

### 2.3. Référence

Comme Référence, on utilise des particules d'or de 40 nm sur lesquelles un anticorps de chèvre anti-Immunoglobuline de lapin a été déposé. Ces particules sont disponibles chez BRITISH BIOCELL (Réf. GAR40), sous la forme de suspensions dans une solution aqueuse de tétraborate de sodium à 2 mM à pH 7,2, stabilisée par de l'azoture de sodium à 0,1%. La densité optique de ces suspensions à 520 nm est d'environ 3 et la concentration en protéine est d'environ 6 µg/ml.

### 2.4. Substances de capture.

### 2.4.1. Première substance de capture.

8 ml d'une solution contenant 213 mg de Gamma Globuline Humaine (G4386, Sigma) et 8,6 mg de chlorhydrate de 2-Iminothiolane (Aldrich, 33056-6) dans du tampon carbonate de sodium (100 mM; pH9) sont incubés pendant une heure à 25°C.

Par ailleurs, 20 ml d'une solution contenant 119,8 mg de céphalosporine-C et 54 mg de sulfosuccinimidyl 4-(N-maléimidométhyl)cyclohexane-1-carboxylate (sSMCC, 22322 Pierce)dans du tampon carbonate de sodium (100 mM, pH9) sont incubés pendant une heure à 25°C.

Les deux solutions préparées précédemment sont ensuite mélangées. On ajuste le pH de la solution résultante à 7,1 par ajout de 3 ml NaH₂PO₄ 500 mM et l'on incube pendant deux heures à 25°C. Le mélange obtenu après incubation est dialysé trois fois contre 1 litre de tampon phosphate de sodium (10 mM, pH 7,5). La solution résultante est filtrée sur un filtre à 0,22 µm, puis elle est aliquotée et congelée à -20°C jusqu'à utilisation.

Lors de l'utilisation, les aliquots sont décongelés, et on y ajoute un colorant alimentaire avant d'effectuer le dépôt sur la membrane afin d'apprécier à tout moment la position exacte du dépôt et la qualité du trait.

La première substance de capture permet de fixer les Identifiants couplés aux Marqueurs libres excédentaires par rapport à la quantité d'antibiotique présent dans l'échantillon.

### 2.4.2. Deuxième substance de capture.

Pour la deuxième substance de capture, on utilise une solution d'immunoglobuline de lapin (Sigma I 5006) à une concentration de 0,5 mg/ml en immunoglobuline dans du tampon phosphate de sodium 10 mM, pH 7,5, gamma globuline humaine 5 mg/ml. Cette seconde substance de capture arrête la Référence lors de la migration du liquide sur le dispositif d'essai.

### 2.5. Dispositif d'essai.

On utilise des dispositifs d'essai contenant les membranes (2), (3) et (4), assemblés selon le mode opératoire décrit à l'exemple 1.1. La membrane (3) de ces dispositifs porte du côté proximal la substance de capture décrite à l'exemple 2.4.1. et du côté distal la substance de capture décrite à l'exemple 2.4.2. Les substances de capture ont été déposées selon le procédé décrit à l'exemple 1.2.

### 2.5.1. Test 1 - Test rapide

On prépare sept échantillons de lait contenant respectivement 0; 2; 4; 5; 6; 8 et 10 ppb de pénicilline G. Chacune de ces solutions est alors analysée de la manière suivante.

On prélève un aliquot de 200 µl d'échantillon de lait et 32,8 µl de solution A préparée à l'exemple 2.2.3. que l'on place dans un tube Eppendorf. Ce mélange est incubé pendant 3 minutes à 47°C. On place ensuite un dispositif d'essai verticalement dans le tube Eppendorf de manière à ce que la première extrémité du dispositif d'essai soit en contact avec le mélange. On laisse migrer le mélange sur le dispositif d'essai tout en incubant l'ensemble pendant 2 minutes à 47°C.

Le Tableau 1 ci-dessous reprend les résultats obtenus pour les 7 échantillons restés. Une valeur d'intensité allant de 0 à 10 est attribuée aux bandes détectées, la valeur 10 étant donnée pour la bande la plus intense et la valeur 0 étant donnée pour la bande la moins intense. Selon cette échelle, on attribue une valeur 6 à la bande de référence. L'intensité du signal observé dans la première bande de détection est inversement proportionnelle à la quantité de pénicilline G présente dans l'échantillon.

**Tableau 1**

| Pénicilline G | Intensité | |
|---|---|---|
| (ppb) | 1ère bande | 2ème bande |
| 0 | 10 | 6 |
| 2 | 8 | 6 |
| 4 | 5 | 6 |
| 5 | 3 | 6 |
| 6 | 2 | 6 |
| 8 | 1 | 6 |
| 10 | 0 | 6 |

Dans cet exemple, lorsque la première bande a une intensité inférieure à celle de la bande de référence, le test est considéré comme positif. Les résultats présentés dans le Tableau 1 montrent que ce test permet de détecter en 5 minutes jusqu'à 4 ppb de pénicilline G dans un échantillon de lait.

### 2.5.2. Test 2 - Test sensible.

On prépare six échantillons de lait contenant respectivement 0; 2; 2,5; 3; 4 et 5 ppb de pénicilline G. Chacune de ces solutions est alors analysée de la manière suivante.

On prélève un aliquot de 200 µl d'échantillon de lait et 32,8 µl de solution B préparée à l'exemple 2.2.3. que l'on place dans un tube Eppendorf. Ce mélange est incubé pendant 5 minutes à 47°C. On place ensuite un dispositif d'essai verticalement dans le tube Eppendorf de manière à ce que la première extrémité du dispositif d'essai soit en contact avec le mélange. On laisse migrer le mélange sur le dispositif d'essai tout en incubant l'ensemble pendant 2 minutes à 47°C.

Le Tableau 2 ci-dessous reprend les résultats obtenus pour les 7 échantillons testés. Une valeur d'intensité allant de 0 à 10 est attribuée aux bandes détectées, la valeur 10 étant donnée pour la bande la plus intense et la valeur 0 étant donnée pour la bande la moins intense. Selon cette échelle, on attribue une valeur 6 à la bande de référence. L'intensité du signal observé dans la première bande de détection est inversement proportionnelle à la quantité de pénicilline G présente dans l'échantillon.

**Tableau 2**

| Pénicilline G | Intensité | |
|---|---|---|
| (ppb) | 1ère bande | 2ème bande |
| 0 | 10 | 6 |
| 2 | 7 | 6 |
| 2,5 | 5 | 6 |
| 3 | 4 | 6 |
| 4 | 1 | 6 |
| 5 | 0 | 6 |

Dans cet exemple, lorsque la première bande a une intensité inférieure à celle de la bande de référence, le test est considéré comme positif. Les résultats présentés dans le Tableau 2 montrent que ce test permet de détecter en 7 minutes jusqu'à 2,5 ppb de pénicilline G dans un échantillon de lait.

### Exemple 3. Détermination d'antibiotiques à noyau β-lactame dans le lait au moyen de BlaR.

Cet exemple illustre la détection dans le lait d'antibiotiques à noyau β-lactame contrôlés par les autorités sanitaires. Le test décrit dans cet exemple utilise le récepteur BlaR-CTD couplé à des billes d'or qui servent d'agents de marquage et utilise un support se présentant sous la forme d'un dispositif d'essai comprenant un support solide sur lequel des membranes sont fixées.

### 3.1. Couplage de BlaR-CTD (Identifiant) aux billes d'or (Marqueur).

### 3.1.1. Biotinylation de BlaR-CTD.

3,79 ml d'une solution d'agent de reconnaissance BlaR-CTD à 6.6 mg/ml sont repris en tampon Phosphate de sodium 20 mM pH 7. On ajoute alors à cette solution de BIaR-CTD 41,71 ml de tampon bicarbonate (0,1 M en bicarbonate de sodium. pH 9) et 2 ml d'une solution d'ester 6-(biotinamido)caproïque de N-hydroxy-succinimide à 2,23 mg/ ml également en tampon bicarbonate. Cette solution est agitée doucement sur agitateur pour tubes sur axe rotatif de type LABINCO (disponible chez VEL, Belgique) à une vitesse de 2 tours/minute pendant 2 heures à température ambiante et à l'abri de la lumière. 2,5 ml d'une solution de tampon Tris 1 M pH 8 sont incubés avec le mélange réactionnel dans les mêmes conditions pendant 30 minutes. La solution ainsi obtenue est dialysée contre du tampon HNM (Hepes 100mM. pH 8, NaCl 100 mM, MgCl₂ 50 mM) pendant 24 heures. De cette manière, on obtient une solution de BIaR-CTD biotinyié que l'on dilue dans du tampon HNM-BSA (Hepes 500 mM, pH 8, NaCl 500 mM, MgCl₂ 250 mM, BSA 10 mg/ml) jusqu'à une concentration de 250 µg de BlaR-CTD biotinylé par ml de tampon. Cette solution est stockée à -20 °C.

### 3.1.2. Agent de marquage.

Comme agent de marquage, on utilise des particules d'or ayant un diamètre de 40 nm, sur lesquelles un anticorps de chèvre anti-biotine a été déposé sous la forme de suspensions dans une solution aqueuse de tétraborate de sodium à 2 mM à pH 7,2, stabilisée par de l'azoture de sodium à 0,1% (disponible chez BRITISH BIOCELL (Réf. GAB40). La densité optique de ces suspensions à 520 nm est d'environ 10 et la concentration en protéine est d'environ 24 µg/ml.

### 3.1.3. Couplage de BlaR-CTD biotinylé aux billes d'or.

La solution de BlaR-CTD biotinylé préparée à l'exemple 3.1.1 est diluée 114,7 fois par du tampon HNM-BSA (Hepes 500 mM, pH 8, NaCl 500 mM, MgCl₂ 250 mM, BSA 10 mg/ml). On mélange à température ambiante 22,5 parties en volume de cette solution diluée de BlaR-CTD biotinylé, 7,5 parties en volume de tampon HNM-BSA, 9,27 parties en volume de suspension de particules d'or servant à marquer BlaR-CTD biotinylé et 6 parties en volume de suspension de particules d'or de référence (voir exemple 3.1.4 ci-dessous).

### 3.1.4. Référence indépendante.

Dans ce test, on utilise également une substance de référence qui fournit une bande dont l'intensité permet de quantifier rapidement la quantité d'antibiotique présent dans l'échantillon.

Pour cela, on utilise des particules d'or de 40 nm sur lesquelles un anticorps de chèvre anti-Immunoglobuline de lapin a été déposé. Ces particules sont disponibles chez BRITISH BIOCELL (Réf. GAR40), sous la forme de suspensions dans une solution aqueuse de tétraborate de sodium à 2 mM à pH 7,2, stabilisée par de l'azoture de sodium à 0,1%. La densité optique de ces suspensions à 520 nm est d'environ 3 et la concentration en protéine est d'environ 6 µg/ml.

### 3.2. Substances de capture

### 3.2.1. Première substance de capture - Antibiotique de référence.

8 ml d'une solution contenant 213 mg de Gamma Globuline Humaine (G4386, Sigma) et 8,6 mg de chlorhydrate de 2-Iminothiolane (Aldrich, 33056-6) dans du tampon carbonate de sodium (100 mM, pH 9) sont incubés pendant une heure à 25°C.

Par ailleurs, 20 ml d'une solution contenant 119,8 mg de céphalosporine-C et 54 mg de sulfosuccinimidyl 4-(N-maléimidométhyl)cyclohexane-1-carboxylate (sSMCC, 22322 Pierce)dans du tampon carbonate de sodium (100 mM, pH 9) sont incubés pendant une heure à 25°C.

Les deux solutions préparées précédemment sont ensuite mélangées. On ajuste le pH de la solution résultante à 7,1 par ajout de 3 ml NaH₂PO₄ 500 mM et l'on incube pendant deux heures à 25°C. Le mélange obtenu après incubation est dialysé trois fois contre 1 litre de tampon phosphate de sodium (10 mM, pH 7,5). La solution résultante est filtrée sur un filtre à 0,22 µm, puis elle est aliquotée et congelée à -20°C jusqu'à utilisation.

Lors de l'utilisation, les aliquotes sont décongelées, et on y ajoute un colorant alimentaire avant d'effectuer le dépôt sur la membrane afin d'apprécier à tout moment la position exacte du dépôt et la qualité du trait.

La première substance de capture permet de fixer BlaR-CTD couplé aux billes d'or présent en excédent par rapport à la quantité d'antibiotique présent dans l'échantillon.

### 3.2.2. Deuxième substance de capture - Substance capable de fixer la référence indépendante.

Pour la deuxième substance de capture, on utilise une solution d'immunoglobuline de lapin (Sigma I 5006) à une concentration de 0,5 mg/ml en immunoglobuline dans du tampon phosphate de sodium 10 mM, pH 7,5, gamma globuline humaine 5 mg/ml. Cette seconde substance de capture arrête la référence indépendante lors de la migration du liquide sur le dispositif d'essai.

### 3.3 Dispositif d'essai.

On utilise des dispositifs d'essai contenant les membranes (2), (3) et (4), assemblés selon le mode opératoire décrit à l'exemple 1.1. La membrane (3) de ces dispositifs porte du côté proximal la substance de capture décrite à l'exemple 3.2.1. et du côté distal la substance de capture décrite à l'exemple 3.2.2. Les substances de capture ont été déposées selon le procédé décrit à l'exemple 1.2.

### 3.4. Détermination des antibiotiques dans le lait.

### 3.4.1. Test en 3 minutes - Test rapide

On prépare 7 échantillons de lait frais contenant respectivement 0;1;2;3;4;5 et 6 ppb de pénicilline G. Chacune de ces solutions est alors analysée de la manière suivante :

On prélève un aliquot de 200 µl d'échantillon de lait et 45.27 µl de solution préparée à l'exemple 3.1.3 que l'on place dans une fiole en verre. Ce mélange est incubé pendant 1 minute à 47°C. On prend un dispositif d'essai que l'on place verticalement dans la fiole en verre de manière à ce que la première extrémité du dispositif d'essai soit en contact avec le mélange et de manière à ce que la seconde extrémité repose sur la paroi de la fiole en verre. On laisse migrer le mélange sur le dispositif d'essai tout en incubant l'ensemble pendant 2 minutes à 47°C.

Le Tableau 1 ci-dessous reprend les résultats obtenus pour les 7 échantillons testés. Une valeur d'intensité allant de 0 à 10 est attribuée aux bandes détectées, la valeur 10 étant donnée pour la bande la plus intense et la valeur 0 étant donnée pour la bande la moins intense. Selon cette échelle, on attribue une valeur de 6 à la bande de référence. L'intensité du signal observé dans la première bande est inversement proportionnelle à la quantité de Pénicilline G présente dans l'échantillon.

**Tableau 1**

| Pénicilline G | Intensité | |
|---|---|---|
| (ppb) | 1ère bande | 2ème bande |
| 0 | 10 | 6 |
| 1 | 9 | 6 |
| 2 | 9 | 6 |
| 3 | 4 | 6 |
| 4 | 0 | 6 |
| 5 | 0 | 6 |
| 6 | 0 | 6 |

Dans cet exemple, lorsque la première bande a une intensité inférieure à celle de la deuxième bande, le test est considéré comme positif. Les résultats présentés dans le Tableau 1 montrent que ce test permet de détecter en 3 minutes moins de 4 ppb de pénicilline G dans un échantillon de lait.

Des essais ont également été réalisés avec d'autres antibiotiques à noyau β-lactame dans les mêmes conditions. Ce test réalisé en 3 minutes permet de détecter l'amoxicilline jusqu'à 5 ppb, l'ampicilline jusqu'à 5 ppb, la cloxacilline à moins de 10 ppb, la dicloxacilline à moins de 20 ppb, l'oxacilline à moins de 20 ppb et la céphapirine jusqu'à 20 ppb dans un échantillon de lait.

### 1.3.2. Test en 5 minutes

On prépare 6 échantillons de lait frais contenant respectivement 0;2;4;6;8 et 10 ppb de cloxacilline. Chacune de ces solutions est alors analysée de la manière suivante.

On prélève un aliquot de 200 µl d'échantillon de lait et 45.27 µl de solution préparée à l'exemple 3.1.3 que l'on place dans une fiole en verre. Ce mélange est incubé pendant 3 minutes à 47°C. On prend un dispositif d'essai que l'on place verticalement dans la fiole en verre de manière à ce que la première extrémité du dispositif d'essai soit en contact avec le mélange et de manière à ce que la seconde extrémité repose sur la paroi de la fiole en verre. On laisse migrer le mélange sur le dispositif d'essai tout en incubant l'ensemble pendant 2 minutes à 47°C.

Le Tableau 2 ci-dessous reprend les résultats obtenus pour les 6 échantillons testés. Une valeur d'intensité allant de 0 à 10 est attribuée aux bandes détectées, la valeur 10 étant donnée pour la bande la plus intense et la valeur 0 étant donnée pour la bande la moins intense. Selon cette échelle, on attribue une valeur de 6 à la bande de référence. L'intensité du signal observé dans la première bande est inversement proportionnelle à la quantité de Cloxacilline présente dans l'échantillon.

**Tableau 2**

| Cloxacilline | Intensité | |
|---|---|---|
| (ppb) | 1ère bande | 2ème bande |
| 0 | 10 | 6 |
| 2 | 6 | 6 |
| 4 | 5 | 6 |
| 6 | 3 | 6 |
| 8 | 3 | 6 |
| 10 | 3 | 6 |

Dans cet exemple, lorsque la première bande a une intensité inférieure à celle de la deuxième bande, le test est considéré comme positif. Les résultats présentés dans le Tableau 2 montrent que ce test permet de détecter en 5 minutes jusqu'à 4 ppb de cloxacilline dans un échantillon de lait.

Des essais ont également été réalisés avec d'autres antibiotiques à noyau β-lactame dans les mèmes conditions. Ce test réalisé en 5 minutes permet de détecter la pénicilline G jusqu'à 3 ppb, l'amoxicilline jusqu'à 4 ppb, l'ampicilline jusqu'à 4 ppb, la dicloxacilline jusqu'à 8 ppb, l'oxacilline jusqu'à 8 ppb. la céphapirine jusqu'à 16 ppb, le ceftiofur jusqu'à 100 ppb, la cefquinone à moins de 20 ppb, la nafcilline jusqu'à 20 ppb et la céfazoline jusqu'à 60 ppb dans un échantillon de lait.

Ce test convient particulièrement bien comme test de tri avant le dépotage des camions de lait dans les silos.

### 1.3.3. Test en 9 minutes

On prépare 6 échantillons de lait frais contenant respectivement 0;4:6;8;10 et 12 ppb de céphapirine. Chacune de ces solutions est alors analysée de la manière suivante.

On prélève un aliquot de 200 µl d'échantillon de lait et 45.27 µl de solution préparée à l'exemple 3.1.3 que l'on place dans une fiole en verre. Ce mélange est incubé pendant 7 minutes à 47°C. On prend un dispositif d'essai que l'on place verticalement dans la fiole en verre de manière à ce que la première extrémité du dispositif d'essai soit en contact avec le mélange et de manière à ce que la seconde extrémité repose sur la paroi de la fiole en verre. On laisse migrer le mélange sur le dispositif d'essai tout en incubant l'ensemble pendant 2 minutes à 47°C.

Le Tableau 3 ci-dessous reprend les résultats obtenus pour les 6 échantillons testés. Une valeur d'intensité allant de 0 à 10 est attribuée aux bandes détectées, la valeur 10 étant donnée pour la bande la plus intense et la valeur 0 étant donnée pour la bande la moins intense. Selon cette échelle, on attribue une valeur de 6 à la bande de référence. L'intensité du signal observé dans la première bande est inversement proportionnelle à la quantité de Céphapirine présente dans l'échantillon.

**Tableau 3**

| Céphapirine | Intensité | |
|---|---|---|
| (ppb) | 1ère bande | 2ème bande |
| 0 | 10 | 6 |
| 4 | 6 | 6 |
| 6 | 5 | 6 |
| 8 | 4 | 6 |
| 10 | 3 | 6 |
| 12 | 3 | 6 |

Dans cet exemple, lorsque la première bande a une intensité inférieure à celle de la deuxième bande, le test est considéré comme positif. Les résultats présentés dans le Tableau 3 montrent que ce test permet de détecter en 9 minutes jusqu'à 6 ppb de céphapirine dans un échantillon de lait.

Des essais ont également été réalisés avec d'autres antibiotiques à noyau β-lactame dans les mêmes conditions. Ce test réalisé en 9 minutes permet de détecter la pénicilline G jusqu'à 3 ppb, l'amoxicilline jusqu'à 4 ppb, l'ampicilline jusqu'à 4 ppb, la cloxacilline jusqu'à 4 ppb, la dicloxacilline à moins de 8 ppb, l'oxacilline à moins de 8 ppb, le ceftiofur jusqu'à 80 ppb, la cefquinone à moins de 20 ppb, la nafcilline à moins de 20 ppb et la céfazoline jusqu'à 45 ppb dans un échantillon de lait.

Ce test réalisé en 9 minutes permet donc de détecter tous les antibiotiques contrôlés actuellement par les autorités européennes, et ce, jusqu'aux limites légales imposées par ces autorités.

### 1.3.4. Test en 20 minutes

On prépare 6 échantillons de lait frais contenant respectivement 0;20;30;40;50 et 60 ppb de ceftiofur. Chacune de ces solutions est alors analysée de la manière suivante.

On prélève un aliquot de 200 µl d'échantillon de lait et 45.27 µl de solution préparée à l'exemple 3.1.3 que l'on place dans une fiole en verre. Ce mélange est incubé pendant 18 minutes à 47°C. On prend un dispositif d'essai que l'on place verticalement dans la fiole en verre de manière à ce que la première extrémité du dispositif d'essai soit en contact avec le mélange et de manière à ce que la seconde extrémité repose sur la paroi de la fiole en verre. On laisse migrer le mélange sur le dispositif d'essai tout en incubant l'ensemble pendant 2 minutes à 47°C.

Le Tableau 4 ci-dessous reprend les résultats obtenus pour les 6 échantillons testés. Une valeur d'intensité allant de 0 à 10 est attribuée aux bandes détectées, la valeur 10 étant donnée pour la bande la plus intense et la valeur 0 étant donnée pour la bande la moins intense. Selon cette échelle, on attribue une valeur de 6 à la bande de référence. L'intensité du signal observé dans la première bande est inversement proportionnelle à la quantité de Ceftiofur présente dans l'échantillon.

**Tableau 4**

| Ceftiofur | Intensité | |
|---|---|---|
| (ppb) | 1ère bande | 2ème bande |
| 0 | 10 | 6 |
| 20 | 6 | 6 |
| 30 | 5 | 6 |
| 40 | 4 | 6 |
| 50 | 3 | 6 |
| 60 | 3 | 6 |

Dans cet exemple, lorsque la première bande a une intensité inférieure à celle de la deuxième bande, le test est considéré comme positif. Les résultats présentés dans le Tableau 4 montrent que ce test permet de détecter en 20 minutes jusqu'à 30 ppb de ceftiofur dans un échantillon de lait.

Ce test en 20 minutes permet donc de détecter en un seul test tous les antibiotiques contrôlés actuellement par les autorités européennes et américaines, et ce, jusqu'aux limites légales imposées par ces autorités.

### Exemple 4. Utilisation d'un dispositif d'essai dans un carter plastique.

On utilise un dispositif d'essai tel que décrit à l'exemple 1.6. Dans ce cas, la mise en contact de l'échantillon avec le dispositif d'essai s'effectue en déposant le mélange incubé dans l'ouverture en forme de cuvette prévue à cet effet.

## Revendications

1. Dispositif d'essai permettant de détecter la présence d'analytes dans un produit laitier liquide par migration capillaire tangentielle dudit produit laitier. comprenant un support solide (1) qui possède une première et une seconde extrémité, sur lequel sont fixées, successivement, en partant de la première extrémité,
- une membrane (2) permettant la purification du liquide analysé,
- une membrane (3) sur laquelle une ou plusieurs substances de capture sont immobilisées, et
- une membrane (4) absorbante,
**caractérisé en ce que** la membrane (2) est une membrane Leukosorb® fabriquée à partir de fibres de polyester non tissées et est capable de retenir les substances présentes dans le produit laitier, qui empêchent la migration sur le dispositif d'essai des analytes éventuellement présents dans le produit laitier et des réactifs de détection utilisés selon la méthode pratiquée, et ce, lors de la migration capillaire tangentielle de l'échantillon après trempage de la première extrémité du dispositif d'essai dans le produit laitier analysé.

2. Dispositif d'essai selon la revendication 1, **caractérisé en ce que** 1a membrane (3) est une membrane en nitro-cellulose à vitesse capillaire élevée.

3. Dispositif d'essai selon les revendications 1 à 2, **caractérisé en ce qu'**il possède en outre une membrane (5) sur laquelle au moins un réactif de détection a été déposé, la membrane (5) étant située avant la membrane (3).

4. Dispositif d'essai selon les revendications 1 à 3, **caractérisé en ce que** les membranes sont recouvertes complètement ou partiellement par un film plastique adhésif (6).

5. Dispositif d'essai selon la revendication 4, **caractérisé en ce que** le film plastique (6) ne recouvre pas les premiers millimètres du dispositif d'essai.

6. Dispositif d'essai selon les revendications 1 à 5, **caractérisé en ce qu'**il est placé dans un boîtier plastique présentant une ouverture en forme de cuvette au-dessus de la membrane (2) et une fenêtre d'ouverture au-dessus de la membrane (3).

7. Procédé pour la détection d'analytes dans un produit laitier liquide, utilisant un dispositif d'essai selon l'une quelconque des revendications 1 à 6 ainsi que des réactifs de détection, comprenant les étapes suivantes:
a) la mise en contact d'un volume déterminé de produit laitier avec le dispositif d'essai, cette mise en contact ayant lieu à la première extrémité du dispositif d'essai,
b) la migration tangentielle par capillarité du produit laitier sur le dispositif d'essai de manière à ce que les analytes et les réactifs de détection éventuellement présents dans le produit laitier passent progressivement sur la membrane (2), puis la membrane (3), et de manière à ce que les constituants du produit laitier qui ne sont pas arrêtés par les membranes (2) et (3) aboutissent dans la membrane (4), et
c) la détermination d'une fixation sur la membrane (3).

8. Procédé selon la revendication 7, **caractérisé en ce que** les réactifs de détection comprennent au moins une substance capable de reconnaître spécifiquement l'analyte ou une substance analogue de cet analyte et au moins un agent de marquage.

9. Procédé selon la revendication 8, **caractérisé en ce qu'**au moins une substance capable de reconnaître spécifiquement l'analyte ou une substance analogue de cet analyte est couplée à au moins un agent de marquage.

10. Procédé selon les revendications 8 ou 9, **caractérisé en ce que** l'agent de marquage est fluorescent, particulaire, radioactif, luminescent ou enzymatique.

11. Procédé selon les revendications 7 à 10, **caractérisé en ce qu'**au moins un réactif de détection est ajouté avant l'étape a).

12. Procédé selon la revendication 11, **caractérisé en ce que** le mélange préparé avant l'étape a) est maintenu dans des conditions d'incubation permettant à l'un des réactifs de détection de former un complexe stable et essentiellement irréversible avec l'analyte ou une substance analogue de cet analyte.

13. Procédé selon les revendications 8 à 12, **caractérisé en ce que** les réactifs de détection comprennent en outre au moins une substance de référence.

14. Procédé selon les revendications 7 à 13, **caractérisé en ce que** l'analyte est une tétracycline, oxytétracycline ou chlortétracycline.

15. Procédé selon les revendications 7 à 13, **caractérisé en ce que** l'analyte est, d'une part une protéine exogène du produit laitier liquide. ou, d'autre part, une protéine endogène du produit laitier.

16. Procédé selon la revendication 15, **caractérisé en ce que** l'analyte est une hormone.

17. Procédé selon la revendication 16, **caractérisé en ce que** l'analyte est la progestérone ou l'hormone de croissance.

18. Procédé selon les revendications 7 à 13, **caractérisé en ce que** l'analyte est un antibiotique au noyau β-lactame.

19. Procédé selon la revendication 7 à 13, **caractérisé en ce que** l'analyte est choisi parmi le groupe constitué par benzylpénicilline ampicilline. amoxicilline, carbénicilline, méthylcilline, cloxacilline, 6-APA. monolactame, axtréoname, mécilliname, céphalexine, céphaloglycine, céphaloridine. nitrocéphine. céfatoxime, céfuroxime, ceftiofur, céphapyrine. 7-ACA.

20. Procédé selon les revendications 7 ou 17, **caractérisé en ce que** la substance capable de reconnaître spécifiquement l'analyte ou une substance analogue de l'analyte est choisie parmi les récepteurs capables de former un complexe stable et essentiellement irréversible avec l'analyte ou une substance analogue de l'analyte et les anticorps monoclonaux ou polyclonaux spécifiques de l'analyte ou d'une substance analogue de l'analyte.

21. Procédé selon la revendication 20, **caractérisé en ce que** la substance capable de reconnaître spécifiquement l'analyte ou une substance analogue de l'analyte est un récepteur obtenu à partir d'un microorganisme sensible aux antibiotiques.

22. Procédé selon la revendication 21, **caractérisé en ce que** la substance capable de reconnaître spécifiquement l'analyte ou une substance analogue de l'analyte est un récepteur obtenu à partir des espèces Bacillus. Streptococcus ou Actinomycètes.

23. Procédé selon les revendications 20, 21 ou 22, **caractérisé en ce que** la substance capable de reconnaitre spécifiquement l'analyte ou une substance analogue de l'analyte est un récepteur sensible aux antibiotiques à noyau β-lactame obtenu à partir de Bacillus licheniformis.

24. Procédé selon la revendication 23, **caractérisé en ce que** le récepteur sensible aux antibiotiques à noyau β-lactame obtenu à partir de Bacillus licheniformis est le récepteur BlaR ou le récepteur BlaR-CTD.

25. Trousse d'essai comprenant un dispositif d'essai selon les revendications 1 à 6.

## Patentansprüche

1. Testvorrichtung, die es ermöglicht, die Gegenwart von Analyten in einem flüssigen Milchprodukt durch tangentiale Kapillarmigration besagten Milchprodukts nachzuweisen, umfassend einen festen Träger (1 ), der ein erstes und ein zweites Ende besitzt, auf dem nacheinander, ausgehend von dem ersten Ende, aufgebracht sind
- eine Membran (2), die die Reinigung der analysierten Flüssigkeit ermöglicht,
- eine Membran (3), auf der eine oder mehrere Fangsubstanzen immobilisiert sind, und
- eine absorbierende Membran (4),
**dadurch gekennzeichnet, dass** die Membran (2) eine Leukosorb®-Membran ist, die aus nicht gewebten Polyesterfasern hergestellt ist und in der Lage ist, die in dem Milchprodukt vorhandenen Substanzen zurückzuhalten, die die Migration der gegebenenfalls in dem Milchprodukt vorhandenen Analyten und der gemäß dem praktizierten Verfahren verwendeten Nachweisreagenzien auf der Testvorrichtung verhindem, und dies bei der tangentialen Kapillarmigration der Probe nach Eintauchen des ersten Endes der Testvorrichtung in das analysierte Milchprodukt.

2. Testvorrichtung gemäß Anspruch 1, **dadurch gekennzeichnet, dass** die Membran (3) eine Membran aus Nitrocellulose mit hoher Kapillargeschwindigkeit ist.

3. Testvorrichtung gemäß den Ansprüchen 1 bis 2, **dadurch gekennzeichnet, dass** sie außerdem eine Membran (5) besitzt, auf der wenigstens ein Nachweisreagenz abgeschieden wurde, wobei die Membran (5) vor der Membran (3) liegt.

4. Testvorrichtung gemäß den Ansprüchen 1 bis 3, **dadurch gekennzeichnet, dass** die Membranen vollständig oder teilweise mit einem Piastikklebeflm (6) bedeckt sind.

5. Testvorrichtung gemäß Anspruch 4, **dadurch gekennzeichnet, dass** der Plastikfilm (6) die ersten Millimeter der Testvorrichtung nicht bedeckt

6. Testvorrichtung gemäß den Ansprüchen 1 bis 5, **dadurch gekennzeichnet, dass** sie in ein Plastikgehäuse eingebracht ist, das eine Öffnung in Form einer Schale oberhalb der Membran (2) und ein Öffnungsfenster oberhalb der Membran (3) aufweist

7. Verfahren für den Nachweis von Analyten in einem flüssigen Milchprodukt, das eine Testvorrichtung gemäß einem der Ansprüche 1 bis 6 sowie Nachweisreagenzien verwendet, das die folgenden Schritte umfasst:
a) das Inkontaktbringen eines bestimmten Milchproduktvolumens mit der Testvorrichtung, wobei dieses Inkontaktbringen am ersten Ende der Testvorrichtung stattfindet,
b) die tangentiale Migration durch Kapillarwirkung des Milchprodukts auf der Testvorrichtung, so dass die Analyten und die Nachweisreagenzien, die gegebenenfalls in dem Milchprodukt vorhanden sind, nach und nach die Membran (2), dann die Membran (3) durchlaufen und so dass die Bestandteile des Milchprodukts, die nicht von den Membranen (2) und (3) angehalten werden, in der Membran (4) enden und
c) die Bestimmung einer Fixierung auf der Membran (3).

8. Verfahren gemäß Anspruch 7, **dadurch gekennzeichnet, dass** die Nachweisreagenzien wenigstens eine Substanz, die in der Lage ist, den Analyten oder eine analoge Substanz dieses Analyten spezifisch zu erkennen, und wenigstens ein Markierungsmittel umfassen.

9. Verfahren gemäß Anspruch 8, **dadurch gekennzeichnet, dass** wenigstens eine Substanz, die in der Lage ist, den Analyten oder eine analoge Substanz dieses Analyten spezifisch zu erkennen, an wenigstens ein Markierungsmittet gekoppelt ist.

10. Verfahren gemäß den Ansprüchen 8 oder 9, **dadurch gekennzeichnet, dass** das Markierungsmittel fluoreszierend, partikelartig, radioaktiv, lumineszierend oder enzymatisch ist.

11. Verfahren gemäß den Ansprüchen 7 bis 10, **dadurch gekennzeichnet, dass** wenigstens ein Nachweisreagenz vor dem Schritt a) hinzugefügt wird.

12. Verfahren gemäß Anspruch 11, **dadurch gekennzeichnet, dass** das vor dem Schritt a) hergestellte Gemisch unter Inkubationsbedingungen gehalten wird, die es einem der Nachweisreagenzien ermöglichen, einen stabilen und im Wesentlichen irreversiblen Komplex mit dem Analyten oder einer analogen Substanz dieses Analyten zu bilden.

13. Verfahren gemäß den Ansprüchen 8 bis 12, **dadurch gekennzeichnet, dass** die Nachweisreagenzien außerdem wenigstens eine Referenzsubstanz umfassen.

14. Verfahren gemäß den Ansprüchen 7 bis 13, **dadurch gekennzeichnet, dass** der Analyt ein Tetracyclin, Oxytetracyclin oder Chlortetracycfin ist.

15. Verfahren gemäß den Ansprüchen 7 bis 13, **dadurch gekennzeichnet, dass** der Analyt einerseits ein exogenes Protein des flüssigen Milchprodukts oder andererseits ein endogenes Protein des Milchprodukts ist.

16. Verfahren gemäß Anspruch 15, **dadurch gekennzeichnet, dass** der Analyt ein Hormon ist.

17. Verfahren gemäß Anspruch 16, **dadurch gekennzeichnet, dass** der Analyt Progesteron oder das Wachstumshormon ist.

18. Verfahren gemäß Anspruch 7 bis 13, **dadurch gekennzeichnet, dass** der Analyt ein Antibiotikum mit β-Laktamkem ist.

19. Verfahren gemäß Anspruch 7 bis 13, **dadurch gekennzeichnet, dass** der Analyt ausgewählt ist aus der Gruppe, die besteht aus Benzylpenicillin, Ampicillin, Amoxicillin, Carbenicillin, Methylcillin, Cloxacillin, 6-APA, Monotaktam, Aztreonam, Mecillinam, Cephatexin, Cephaloglycin, Cephaloridin, Nitrocephin, Cefatoxim, Cefuroxim, Ceftiofur, Cephapyrin, 7-ACA

20. Verfahren gemäß den Ansprüchen 7 oder 17, **dadurch gekennzeichnet, dass** die Substanz, die in der Lage ist, den Analyten oder eine analoge Substanz des Analyten spezifisch zu erkennen, ausgewählt ist unter den Rezeptoren, die in der Lage sind, einen stabilen und im Wesentlichen irreversiblen Komplex mit dem Analyten oder einer analogen Substanz des Analyten zu bilden, und den spezifischen monoklonalen oder polyklonalen Antikörpern des Analyten oder einer analogen Substanz des Analyten.

21. Verfahren gemäß Anspruch 20, **dadurch gekennzeichnet, dass** die Substanz, die in der Lage ist, den Analyten oder eine analoge Substanz des Analyten spezifisch zu erkennen, ein Rezeptor ist, der aus einem Mikroorganismus erhalten wird, der gegen die Antibiotika empfindlich ist.

22. Verfahren gemäß Anspruch 21, **dadurch gekennzeichnet, dass** die Substanz, die in der Lage ist, den Analyten oder eine analoge Substanz des Analyten spezifisch zu erkennen, ein Rezeptor ist, der aus den Spezies Bacillus, Streptococcus oder Actinomycetes erhalten wird.

23. Verfahren gemäß den Ansprüchen 20, 21 oder 22, **dadurch gekennzeichnet, dass** die Substanz, die in der Lage ist, den Analyten oder eine analoge Substanz des Analyten spezifisch zu erkennen, ein gegen die Antibiotika mit β-Laktamkem empfindlicher Rezeptor ist, der aus Bacillus licheniformis erhalten wird.

24. Verfahren gemäß Anspruch 23, **dadurch gekennzeichnet, dass** der gegen die Antibiotika mit β-Laktamkem empfindliche Rezeptor, der aus Bacillus licheniformis erhalten wird, der Rezeptor BlaR oder der Rezeptor BlaR-CTD ist.

25. Testkit, umfassend eine Testvorrichtung gemäß den Ansprüchen 1 bis 6.

## Claims

1. Test device which allows detection of the presence of analytes in a liquid dairy product by tangential capillary migration of the said dairy product, comprising a solid support (1) which has a first and a second end on to which are fixed, successively, starting from the first end,
- a membrane (2) which allows purification of the liquid analysed,
- a membrane (3), on to which one or more trapping substances are immobilized, and
- an absorbent membrane (4),
**characterized in that** the membrane (2) is a Leukosorb® membrane produced from nonwoven polyester fibres and is capable of retaining the substances present in the dairy product which prevent migration on the test device of any analytes present in the dairy product and of the detection reagents used, according to the method carried out, during tangential capillary migration of the sample after steeping of the first end of the test device in the dairy product analysed.

2. Test device according to claim 1, **characterized in that** the membrane (3) is a nitrocellulose membrane of high capillary speed.

3. Test device according to claims 1 to 2, **characterized in that** it also has a membrane (5) on to which at least one detection reagent has been deposited, the membrane (5) being located before the membrane (3).

4. Test device according to claims 1 to 3, **characterized in that** the membranes are completely or partly covered by an adhesive film of plastic (6).

5. Test device according to claim 4, **characterized in that** the film of plastic (6) does not cover the first millimetres of the test device.

6. Test device according to claims 1 to 5, **characterized in that** it is positioned in a box of plastic having an opening in the form of a cell above the membrane (2) and a window opening above the membrane (3).

7. Process for the detection of analytes in a liquid dairy product using a test device according to any one of claims 1 to 6 and detection reagents, comprising the following stages:
a) bringing a given volume of dairy product into contact with the test device, this bringing into contact taking place at the first end of the test device,
b) tangential migration by capillarity of the dairy product on the test device in a manner such that the analytes and the detection reagents which may be present in the dairy product pass progressively over the membrane (2) and then the membrane (3) in a manner such that the constituents of the dairy product which are not stopped by the membranes (2) and (3) arrive at the membrane (4), and
c) determination of fixing on the membrane (3).

8. Process according to claim 7, **characterized in that** the detection reagents comprise at least one substance capable of recognizing specifically the analyte or a substance analogous to this analyte and at least one labelling agent.

9. Process according to claim 8, **characterized in that** at least one substance capable of recognizing specifically the analyte or a substance analogous to this analyte is coupled to at least one labelling agent.

10. Process according to claims 8 or 9, **characterized in that** the labelling agent is fluorescent, particulate, radioactive, luminescent or enzymatic.

11. Process according to claims 7 to 10, **characterized in that** at least one detection reagent is added before stage a).

12. Process according to claim 11, **characterized in that** the mixture prepared before stage a) is kept under incubation conditions which allow one of the detection reagents to form a stable and essentially irreversible complex with the analyte or a substance analogous to this analyte.

13. Process according to claims 8 to 12, **characterized in that** the detection reagents also comprise at least one reference substance.

14. Process according to claims 7 to 13, **characterized in that** the analyte is a tetracycline, oxytetracycline or chlortetracycline.

15. Process according to claims 7 to 13, **characterized in that** the analyte is on the one hand an exogenous protein of the liquid dairy product or on the other hand an endogenous protein of the dairy product.

16. Process according to claim 15, **characterized in that** the analyte is a hormone.

17. Process according to claim 16, **characterized in that** the analyte is progesterone or a growth hormone.

18. Process according to claims 7 to 13, **characterized in that** the analyte is an antibiotic having a β-lactam ring.

19. Process according to claim 7 to 13, **characterized in that** the analyte is chosen from the group consisting of benzylpenicillin, ampicillin, amoxicillin, carbenicillin, methylcillin, cloxacillin, 6-APA, monolactam, aztreonam, mecillinam, cephalexin, cephaloglycin, cephaloridine, nitrocephin, cefatoxime, cefuroxim, ceftiofur, cefapirin and 7-ACA.

20. Process according to claims 7 or 17, **characterized in that** the substance capable of recognizing specifically the analyte or a substance analogous to the analyte is chosen from the receptors capable of forming a stable and essentially irreversible complex with the analyte or a substance analogous to the analyte and monoclonal or polyclonal antibodies specific to the analyte or a substance analogous to the analyte.

21. Process according to claim 20, **characterized in that** the substance capable of recognizing specifically the analyte or a substance analogous to the analyte is a receptor obtained from a microorganism which is sensitive to antibiotics.

22. Process according to claim 21, **characterized in that** the substance capable of recognizing specifically the analyte or a substance analogous to the analyte is a receptor obtained from the species Bacillus, Streptococcus or Actinomycetes.

23. Process according to claims 20, 21 or 22, **characterized in that** the substance capable of recognizing specifically the analyte or a substance analogous to the analyte is a receptor which is sensitive to antibiotics having a β-lactam ring obtained from Bacillus licheniformis.

24. Process according to claim 23, **characterized in that** the receptor which is sensitive to antibiotics having a β-lactam ring obtained from Bacillus licheniformis is the receptor BlaR or the receptor BlaR-CTD.

25. Test kit comprising a test device according to claims 1 to 6.
